# FASCICULE DE BREVET EUROPEEN

(11) **EP 1 676 560 B1**
(45) Date de publication et mention de la délivrance du brevet: **24.09.2008**
(21) Numéro de dépôt: 05025949.8
(22) Date de dépôt: 29.11.2005
(51) Int. Cl.: A61K 8/03, A61K 8/06, A61K 8/899, A61Q 1/14, A61Q 19/00

(54) **Composition biphase contenant un tensioactif de type sel de sodium d'esters de diméthicone copolyol et d'acide sulfosuccinique, et ses utilisations dans le domaine cosmétique**
Zweiphasige Zusammensetzung enthaltend ein Natriumsalz eines Esters des Dimethiconcopolyols mit Sulfobernsteinsäure, und ihre Verwendung in der Kosmetik
Biphasic composition containing a sodium salt of esters of dimethicone copolyol and sulfosuccinic acid type surfactant, and its uses in the cosmetic field

(30) Priorité: 03.01.2005 FR 0550009
(43) Date de publication de la demande: 05.07.2006
(73) Titulaire: L'ORÉAL, 75008 Paris (FR)
(72) Inventeur: Fonolla Moreno, Angeles, 75013 Paris (FR)
(74) Mandataire: Rasson, Catherine

(56) Documents cités:
- DE-C1- 3 627 313
- US-A1- 2002 010 110

## Description

La présente invention a pour objet une composition pour application topique, constituée de deux phases distinctes, une phase aqueuse et une émulsion, ces phases se mélangeant facilement par agitation et se déphasant facilement après arrêt de l'agitation. La présente invention a aussi pour objet l'utilisation de la dite composition dans le domaine cosmétique ou dermatologique, et notamment pour le démaquillage, le nettoyage et/ou le soin de la peau, des lèvres et/ou des yeux, et/ou pour le soin des cheveux.

Les compositions conventionnelles démaquillantes peuvent se présenter sous différentes formes, par exemple sous forme de laits démaquillants plus ou moins fluides contenant eau, huiles et tensioactifs, de gels aqueux moussants, ou de compositions huileuses. Chacune de ces galéniques présente des avantages et des inconvénients. Ainsi, les laits démaquillants présentent l'avantage de donner une sensation agréable lors de l'application sur la peau, d'hydrater la peau et de respecter la barrière cutanée, mais ils présentent l'inconvénient de donner un toucher plus ou moins gras, sans fraîcheur, et de laisser un film sur la peau. Les gels aqueux présentent l'avantage de donner une bonne sensation de fraîcheur, une sensation de propreté immédiate et persistante et un toucher non gras, mais ils présentent l'inconvénient de donner aussi une sensation de tiraillement (sticky quiet), et de pouvoir dessécher la peau si l'utilisation est trop fréquente. Quant aux huiles démaquillantes, elles ont l'avantage d'hydrater la peau, de respecter la barrière cutanée et de rétablir l'équilibre lipidique de la peau, tout en étant très efficaces pour le démaquillage, mais elles manquent de fraîcheur, donnent un toucher gras et laissent un film gras sur la peau.

Pour éviter les inconvénients de l'art antérieur, il a été proposé des compositions constituées de deux phases distinctes, notamment d'une phase aqueuse et d'une phase huileuse distinctes et non émulsionnées l'une dans l'autre au repos, qui sont généralement désignées sous le terme de "composition biphase". Elles se distinguent des émulsions par le fait qu'au repos, les deux phases sont distinctes au lieu d'être émulsionnées l'une dans l'autre. L'utilisation de ces compositions biphase nécessite une agitation préalable afin de former une émulsion extemporanée, celle-ci devant être de qualité et de stabilité suffisantes pour permettre une application homogène des deux phases sur la peau ou la matière kératinique où elle est appliquée. Au repos, lesdites phases doivent se séparer rapidement et retrouver leur état initial, ce phénomène étant plus connu sous le terme de "déphasage".

Des compositions biphases ont déjà été décrites, par exemple dans les documents EP-A-370856 et EP-A-603080, notamment pour le démaquillage des yeux. Ces compositions présentent l'avantage d'être plus fraîches à l'application que les laits et les compositions huileuses, tout en étant efficaces, et de ne donner aucune sensation de tiraillement ou d'irritation, tout en conférant à la peau de la douceur. Toutefois, elles présentent l'inconvénient de ne pas donner toujours une impression de fraîcheur suffisante, et de ne pas s'éliminer facilement.

Il subsiste donc le besoin d'une composition biphase qui ait d'excellentes qualités cosmétiques (douceur, fraîcheur, tolérance), et une très bonne efficacité aussi bien pour le démaquillage que pour le soin.

De manière surprenante, la demanderesse a trouvé que l'utilisation de tensioactifs particuliers dans une composition biphase comportant une phase supérieure émulsionnée et une phase inférieure aqueuse permettait d'obtenir une composition biphase ayant les qualités recherchées, c'est-à-dire très efficace mais aussi très douce et fraîche à l'application. Ces tensioactifs présentent l'avantage d'être très bien tolérés y compris pour une application sur les yeux qui sont particulièrement sensibles aux composés susceptibles d'être agressifs.

Ainsi, l'invention a pour objet une composition pour application topique, constituée de deux phases distinctes l'une au-dessus de l'autre, comprenant (1) une phase aqueuse inférieure, (2) une phase supérieure constituée d'une émulsion huile-dans-eau, (3) au moins un tensioactif anionique choisi parmi les sels de sodium d'esters de diméthicone copolyol et d'acide sulfosuccinique et où la phase aqueuse inférieure et la phase aqueuse de l'émulsion huile-dans-eau ont la même composition.

Les sels de sodium d'esters de diméthicone copolyol et d'acide sulfosuccinique sont des tensioactifs connus, déjà utilisés dans des compositions cosmétiques, mais il n'a jamais été décrit leur utilisation pour obtenir des compositions se présentant sous forme de deux phases distinctes qui se trouvent l'une sur l'autre dans un même récipient, phases qui sont agitées au moment de l'utilisation et qui se séparent de nouveau au repos. Ainsi, le document US-A-2002/0010110 décrit une composition extrudable comprenant deux phases qui se trouvent l'une à côté de l'autre, séparées par une couche de démarcation, et qui sont extrudées ensemble du récipient les contenant par une sortie commune, une des phases étant une phase isotrope et l'autre étant une phase lamellaire. Parmi les tensioactifs décrits, se trouve le dimethicone copolyol sulfosuccinate. Toutefois, les compositions décrites dans ce document ne contiennent pas forcément le dimethicone copolyol sulfosuccinate comme tensioactif, alors que, dans la présente demande, seuls les sels de sodium de diméthicone copolyol et d'acide sulfosuccinique permettent d'atteindre le but de l'invention. En outre, dans ce document, une des phases est une phase lamellaire et les compositions décrites contiennent globalement une quantité importante de tensioactifs, alors que, dans la présente invention, la phase supérieure de la composition biphase qui est sous forme d'émulsion ne constitue pas une phase lamellaire, et la teneur totale en tensioactifs est d'au plus 20% en poids par rapport au poids total de la composition biphase. Par ailleurs, dans les compositions décrites dans ce document, les deux phases ne se trouvent pas l'une au dessus de l'autre, et quand on les mélange et qu'on les laisse ensuite reposer, elles ne se déphasent pas en deux phases distinctes. Ce document décrit donc une composition différente de celle revendiquée.

La composition selon l'invention étant destinée à une application topique, elle contient un milieu physiologiquement acceptable, c'est-à-dire compatible avec la peau, les muqueuses, les cheveux et le cuir chevelu. La composition peut être notamment une composition cosmétique ou dermatologique.

Le tensioactif anionique utilisé selon l'invention présente l'avantage non seulement d'être efficace pour un démaquillage en douceur mais aussi de permettre l'obtention d'une composition biphase comportant une phase sous forme d'émulsion, et ce sans l'intervention d'un autre émulsionnant.

La composition selon l'invention comprend au moins une phase aqueuse et une émulsion constituant une phase distincte de la phase aqueuse. Ces deux phases sont distinctes, c'est-à-dire qu'elles sont visibles l'une au-dessus de l'autre au repos et qu'elles apparaissent donc visuellement comme non mélangées quand elles sont au repos. La phase aqueuse constitue la phase inférieure tandis que l'émulsion constitue la phase supérieure. En outre, selon une caractéristique de l'invention, l'émulsion n'est pas transparente, mais elle a l'aspect d'un lait. La phase aqueuse est généralement transparente. Les deux phases peuvent être ou non colorées.

L'émulsion constituant la phase supérieure doit être considérée comme formant une seule phase dans la composition biphase de l'invention, bien qu'il soit connu que les émulsions sont formées de deux phases non miscibles dispersées l'une dans l'autre. On peut la considérer comme formant une seule phase de la composition revendiquée car il s'agit d'une émulsion qui reste stable dans le temps, c'est-à-dire plusieurs mois et même plusieurs années, sans que ne se produisent ni séparation de phases ni décantation, et cette émulsion constitue une phase de la composition biphase selon l'invention tandis que la phase aqueuse constitue l'autre phase, l'émulsion et la phase aqueuse étant disposées l'une au-dessus de l'autre. Lors de l'utilisation, elles sont agitées pour donner une émulsion homogène qui ne reste sous forme d'émulsion homogène que quelque temps, généralement quelques minutes ou tout au plus quelques heures, et qui se sépare de nouveau au repos en une phase aqueuse et une phase d'émulsion.

Le rapport pondéral entre la phase aqueuse et l'émulsion peut aller par exemple de 25/75 à 90/10, de préférence 30/70 à 70/30 et mieux de 40/60 à 60/40.

La quantité de constituants aqueux comprenant la phase aqueuse inférieure et la phase aqueuse de l'émulsion représente généralement de 10 à 90 % en poids, de préférence de 20 à 85 % en poids, mieux de 30 à 80 % en poids et encore mieux de 40 à 70 % en poids par rapport au poids total de la composition. Par ailleurs, la phase aqueuse inférieure et la phase aqueuse de l'émulsion ont la même composition.

### Tensioactif anionique

La composition selon l'invention contient au moins un tensioactif anionique choisi parmi les sels de sodium d'esters de diméthicone copolyol et d'acide sulfosuccinique.

Il s'agit notamment de composés de formule (I) : 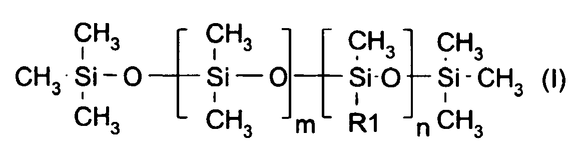 dans laquelle R₁ peut représenter : 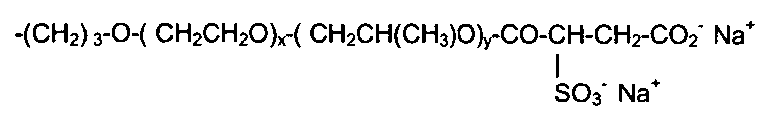 Ou bien : 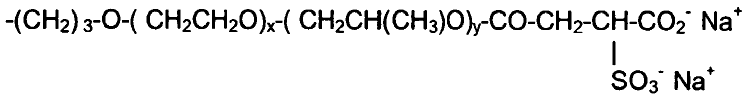 où m, n, x et y sont des nombres entiers qui varient suivant le diméthicone copolyol utilisé : m et n peuvent aller par exemple de 2 à 100 et mieux de 2 à 50 ; x peut aller par exemple de 1 à 50 et mieux de 2 à 40, y peut aller par exemple de 0 à 50.

Suivant un mode préféré de réalisation de l'invention, le diméthicone copolyol utilisé ne comprend que des groupes oxyéthylénés, c'est-à-dire que dans la formule de R1, y=0.

De façon préférée, le tensioactif choisi parmi les sels de sodium d'esters de diméthicone copolyol et d'acide sulfosuccinique est le PEG-12 dimethicone sulfosuccinate de disodium (nom CTFA: Disodium PEG-12 Dimethicone sulfosuccinate), composé de formule (I) où m = 8, n = 3, y=0 et x= 12. Comme Disodium PEG-12 Dimethicone sulfosuccinate, on peut citer en particulier ceux commercialisés par la société MCINTYRE soit à 50 % en solution aqueuse sous la dénomination MACKANATE DC 50, soit à 30 % en solution aqueuse sous la dénomination MACKANATE DC 30.

La quantité de tensioactif choisi parmi les sels de sodium d'esters de diméthicone copolyol et d'acide sulfosuccinique, en quantité de matière active, est variable notamment selon les proportions relatives globales de constituants huileux et de constituants aqueux et selon l'utilisation finale de la composition. En effet si la composition constitue un produit de soin de la peau, elle contiendra moins de tensioactif anionique dimethicone sulfosuccinate que si elle constitue un produit de démaquillage, où ce tensioactif jouera aussi un rôle de démaquillant.

Cette quantité de tensioactif anionique, en matière active, peut aller par exemple de 0,1 à 20 % en poids, de préférence de 0,2 à 15 % en poids, mieux de 0,2 à 10 % en poids par rapport au poids total de la composition. De manière plus spécifique, la quantité en matière active dans un produit démaquillant peut aller par exemple de 0,5 à 20 %, de préférence de 1 à 15 % et mieux de 1 à 10 % en poids par rapport au poids total de la composition, et la quantité en matière active dans un produit de soin peut aller par exemple de 0,2 à 20 %, de préférence de 0,5 à 15 et mieux de 1 à 10 % en poids par rapport au poids total de la composition.

De manière préférée, la quantité totale de tensioactifs est d'au plus 15 % en poids par rapport au poids total de la composition.

Le tensioactif anionique utilisé selon l'invention est généralement introduit dans la phase aqueuse.

Selon un mode préféré de réalisation de l'invention, la composition contient comme seul tensioactif, les sels de sodium d'esters de diméthicone copolyol et d'acide sulfosuccinique Elle est en particulier exempte d'acide gras, d'alcool gras, d'ester d'acide gras et de polyol, et de trihydroxystearine.

### Phase aqueuse

La phase aqueuse de la composition selon l'invention constitue la phase inférieure. Par ailleurs, l'émulsion qui constitue la phase supérieure de la composition biphase est une émulsion huile-dans-eau (phase huileuse dispersée dans la phase aqueuse) et comporte donc une phase aqueuse externe qui contient de l'eau et éventuellement certains composés hydrosolubles bien que ceux-ci puissent se trouver partiellement ou totalement dans la phase aqueuse inférieure. La phase aqueuse de l'émulsion et la phase aqueuse supérieure du biphase constituent la quantité globale de phase aqueuse et ont la même composition.

Au cours de la fabrication de la composition biphase, les constituants aqueux et les constituants huileux vont être mélangés et une partie de la phase aqueuse va devenir phase externe de l'émulsion tandis que l'autre partie constituera la phase aqueuse inférieure. On entend donc ci-dessous par « phase aqueuse » aussi bien la phase aqueuse inférieure du biphase que la phase aqueuse de l'émulsion H/E.

Comme indiqué ci-dessus, la quantité globale de constituants aqueux dans la composition selon l'invention peut aller par exemple de 10 à 90 % en poids, de préférence de 20 à 80 % en poids, mieux de 30 à 70 % en poids et encore mieux de 40 à 60 % en poids par rapport au poids total de la composition.

La phase aqueuse comprend de l'eau et tout additif hydrosoluble ou hydrodispersible. L'eau utilisée peut être de l'eau déminéralisée stérile et/ou une eau florale telle que de l'eau de rose, de l'eau de bleuet, de l'eau de camomille ou de l'eau de tilleul, et/ou une eau thermale ou minérale naturelle, comme par exemple : l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene. La phase aqueuse peut comprendre aussi de l'eau thermale reconstituée, c'est-à-dire une eau contenant des oligoéléments tels que zinc, cuivre, magnésium, etc.., reconstituant les caractéristiques d'une eau thermale.

Comme additifs hydrosolubles, on peut citer notamment les polyols tels que la glycérine et les glycols tels que le butylène glycol, l'hexylène glycol, les polyéthylène glycols et le polypropylène glycol. Les polyols peuvent être présents en une quantité allant de 0 à 25 % en poids, de préférence de 3 à 20 %, mieux de 3 à 15 % en poids et encore mieux de 5 à 10 % en poids par rapport au poids total de la composition. Selon un mode préféré de réalisation de l'invention, la composition contient au moins un polyol, de préférence la glycérine ou Le butylène glycol ou leurs mélanges.

Comme additifs hydrosolubles, on peut citer aussi les alcools primaires en C₂-C₈, et notamment l'éthanol. Selon un mode particulier de réalisation de l'invention, la composition est de préférence pratiquement exempte d'éthanol. On entend ici par "pratiquement exempte d'éthanol", une composition contenant moins de 5 % en poids et de préférence moins de 2 % en poids d'éthanol par rapport au poids total de la composition.

### Emulsion

L'émulsion huile-dans-eau (H/E) constitue la phase supérieure de l'émulsion, et elle comprend une phase aqueuse externe et une phase huileuse comportant les huiles et autres corps gras ou additifs lipophiles, dispersée dans la phase aqueuse. L'émulsion représente généralement de 10 à 75 %, de préférence de 30 à 70 % en poids, et mieux de 40 à 60 % en poids par rapport au poids total de la composition.

L'émulsion H/E de la composition selon l'invention comprend une phase huileuse dispersée dans une phase aqueuse.

La composition selon l'invention peut contenir une ou plusieurs huiles dans la phase huileuse de l'émulsion constituant la phase supérieure de la composition biphase. Ces huiles peuvent être des huiles minérales, végétales ou synthétiques ou encore des huiles de silicone. Il peut y avoir en outre dans la phase huileuse, des additifs liposolubles ou lipodispersibles.

Selon un mode préféré de réalisation de l'invention, la composition comprend une ou plusieurs huiles choisies parmi les huiles hydrocarbonées d'origine minérale ou synthétique et les huiles de silicone. Plus particulièrement, la composition contient avantageusement une ou plusieurs huiles volatiles choisies parmi les huiles hydrocarbonées volatiles d'origine minérale ou synthétique et les huiles de silicone volatiles.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor; elle peut contenir des groupes ester, éther, amine, amide.

Comme huiles hydrocarbonées volatiles d'origine minérale ou synthétique, on peut citer les iso-alcanes (appelées aussi isoparaffines) en C₈-C₁₆, tels que l'isododécane, l'isodécane, l'isohexadécane, comme par exemple les iso-alcanes vendus sous les noms commerciaux Isopar par la société Exxon Chemical ou les huiles vendues sous les noms commerciaux Permethyl par la société Presperse ; et leurs mélanges.

Comme huiles hydrocarbonées non-volatiles d'origine minérale ou synthétique, on peut citer l'huile de vaseline, le polyisobutène hydrogéné tel que l'huile de Parléam® ; et leurs mélanges.

Par huile de silicone, on entend une huile contenant au moins un atome de silicium, et notamment contenant des groupes Si-O. L'huile de silicone peut être choisie parmi les huiles de silicone non volatiles, les huiles de silicone volatiles et leurs mélanges.

Les huiles de silicone volatiles utilisables dans l'invention peuvent être choisies parmi les huiles de silicone ayant un point éclair allant de 40°C à 102°C, de préférence ayant un point éclair supérieur à 55°C et inférieur ou égal à 95°C, et préférentiellement allant de 65°C à 95°C. Comme huiles de silicone volatiles, on peut citer les huiles de silicones linéaires ou cycliques ayant de 2 à 7 atomes de silicium, ces silicones comportant éventuellement des groupes alkyle ou alkoxy ayant de 1 à 10 atomes de carbone. Comme exemples d'huiles de silicone volatiles, on peut citer notamment les cyclopolydiméthylsiloxanes (nom INCI : cyclomethicone), telles que le cyclopentasiloxane, le cyclohexasiloxane, l'octaméthylcyclotétrasiloxane, le décaméthylcyclopentasiloxane, le dodéca-méthylcyclohexasiloxane ; les silicones linéaires telles que l'heptaméthylhexyl-trisiloxane, l'heptaméthyloctyl-trisiloxane, l'hexaméthyl-disiloxane, l'octaméthyl-trisiloxane, le décaméthyltétrasiloxane, le dodécaméthyl pentasiloxane ; et leurs mélanges.

L'huile de silicone non volatile utilisable dans l'invention peut être choisie parmi les polydiméthylsiloxanes (PDMS), et les polyméthylsiloxanes phénylés tels que les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ; les polysiloxanes modifiés par des acides gras, des alcools gras ou des polyoxyalkylènes, et leurs mélanges.

De manière préférée, la composition selon l'invention contient au moins une huile volatile choisie parmi les iso-alcanes, les huiles de silicone volatiles, et leurs mélanges. Selon un autre mode préférentiel de réalisation de l'invention, la phase huileuse contient au moins un iso-alcane choisi parmi l'isododécane et l'isohexadécane, et au moins une huile de silicone volatile.

Par ailleurs, la phase huileuse peut contenir une ou plusieurs autres huiles volatiles ou non, choisies parmi les huiles hydrocarbonées d'origine animale ou végétale, les esters et éthers de synthèse, les alcools gras, les huiles fluorées et leurs mélanges.

Par huile fluorée, on entend une huile contenant au moins un atome de fluor.

On peut citer par exemple comme huiles utilisables dans la composition de l'invention :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'amande d'abricot, de macadamia, d'arara, de coriandre, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le düsostéaryl-malate, le citrate de triisocétyle, les heptanoates, octanoates, décanoates d'alcools gras ; les esters de polyol, comme le dioctanoate de propylène glycol, le diheptanoate de néopentylglycol et le düsononanoate de diéthylèneglycol ; et les esters du pentaérythritol comme le tétraisostéarate de pentaérythrytyle.

Les huiles peuvent éventuellement être constituées uniquement d'huiles volatiles.

La quantité de phase huileuse de l'émulsion de la composition selon l'invention peut varier dans une large mesure selon la finalité de la composition. Elle peut aller par exemple de 5 à 50 % en poids, de préférence de 10 à 40 % en poids et mieux de 15 à 35 % en poids par rapport au poids total de la composition.

### Additifs

La composition selon l'invention peut contenir des adjuvants ou additifs cosmétiques conventionnels qui se trouveront dans l'une ou l'autre phase selon leur nature hydrophile ou lipophile, tels que par exemple des parfums, des agents conservateurs et bactéricides, des colorants, des agents adoucissants, des tampons, des humectants, des filtres U.V. (ou filtres solaires), des électrolytes tel que le chlorure de sodium comme indiqué plus haut, ou un ajusteur de pH (par exemple acide citrique ou hydroxyde de sodium), des agents facilitant la séparation de phase, des polymères, et leurs mélanges.

Comme conservateurs, on peut utiliser tout conservateur habituellement utilisé dans les domaines considérés, tels que par exemple les parabens, le gluconate de chlorhexidine et le chlorhydrate de polyhexaméthylène biguanide (nom CTFA Polyaminopropyl biguanide). Selon un mode préféré de réalisation de l'invention, la composition contient du chlorhydrate de polyhexaméthylène biguanide, seul ou en mélange avec d'autres conservateurs.

Comme bactéricide, on peut par exemple utiliser un mono(C₃-C₉)alkyl-ou (C₃₋C₉)alcényléther de glycérol dont la fabrication est décrite dans la littérature, en particulier dans E. Baer, H.O.L. Fischer - J. Biol. Chem. 140-397-1941. Parmi ces mono(C₃-C₉)alkyl-ou (C₃-C₉)alcényléthers de glycérol, on utilise de préférence le 3-[(2-ethylhexyl)oxy]-1,2-propanediol, le 3-[(heptyl)oxy]-1,2-propanediol, le 3-[(octyl)oxy]-1,2-propanediol et le 3-[(allyl)oxy]-1,2-propanediol. Un mono(C₃₋C₉)alkyléther de glycérol plus particulièrement préféré selon la présente invention est le 3-[(2-ethylhexyl)oxy]-1,2-propanediol, vendu par la société SCHULKE & MAYR G.m.b.H. sous la dénomination commerciale SENSIVA SC 50 (nom INCI : Ethylhexylglycerin).

Parmi les agents adoucissants, on peut en particulier citer l'allantoïne, le bisabollol, les planctons, et certains extraits de plantes comme les extraits de rose et les extraits de mélilot.

Le ou les actifs pouvant être présents dépendent du but final de la composition. Comme actifs utilisables dans la composition de l'invention, notamment quand il s'agit d'une composition de soin de la peau, on peut citer par exemple, les enzymes (par exemple lactoperoxydase, lipase, protéase, phospholipase, cellulases) ; les flavonoïdes tels que les isoflavones ; les agents hydratants tels que les hydrolysats de protéines ; le hyaluronate de sodium ; les anti-inflammatoires ; les oligomères procyannidoliques ; les vitamines comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; l'urée ; la caféine ; les dépigmentants tels que l'acide kojique, l'hydroquinone et l'acide caféique ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; l'hydrocortisone ; la mélatonine ; les extraits d'algues, de champignons, de végétaux, de levures, de bactéries ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents tenseurs ; les céramides ; les huiles essentielles ; et leurs mélanges ; et tout actif approprié pour le but final de la composition.

Les filtres U.V. peuvent être présents dans la composition selon l'invention, notamment quand elle est destinée à une protection solaire. Ces filtres peuvent être notamment des filtres organiques, et ils peuvent être présents en une quantité en matière active allant de 0,01 à 20 % en poids de matière active, de préférence de 0,1 à 15 % en poids, et mieux 0,2 à 10 % en poids par rapport au poids total de la composition.

Comme exemples de filtres organiques, actifs dans l'UV-A et/ou l'UV-B, pouvant être ajoutés dans la composition de l'invention, on peut citer par exemple, les dérivés à fonction sulfonique tels que les dérivés sulfonés ou sulfonatés du benzylidène camphre, de la benzophénone ou du phénylbenzimidazole, plus particulièrement les dérivés du benzylidène camphre, comme l'acide benzène 1,4 [di(3-méthylidène-campho 10-sulfonique)], (nom INCI : Terephthalylidene Dicamphor Sulfonic Acid) fabriqué sous le nom « MEXORYL SX » par la société CHIMEX. l'acide 4'-sulfo 3-benzylidènecamphre (nom INCI : Benzylidene Camphor Sulfonic Acid), fabriqué sous le nom « MEXORYL SL» par la société CHIMEX, l'acide 2-[4-(camphométhylidène) phényl] benzimidazole-5-sulfonique, l'acide phénylbenzimidazole sulfonique (nom INCI : Phenylbenzimidazole Sulfonic Acid), commercialisé sous le nom EUSOLEX 232 par la société MERCK ; les dérivés de l'acide para-aminobenzoique ; les dérivés salicyliques tels que le salicylate d'éthyl hexyle vendu sous le nom commercial NEO HELIOPAN OS par Haarmann et Reimer ; les dérivés du dibenzoylméthane tels que le Butyl Methoxydibenzoylmethane vendu notamment sous le nom commercial PARSOL 1789 par Hoffmann La Roche ; les dérivés cinnamiques tels que l'éthylhexyl Methoxycinnamate vendu notamment sous le nom commercial PARSOL MCX par Hoffmann La Roche ; les dérivés de β,β'-diphénylacrylate tels que l'octocrylene (α-cyano-β,β-diphénylacrylate de 2-éthylhexyle) vendu sous le nom commercial UVINUL N539 par la société BASF ; les dérivés de la benzophénone tels que la Benzophenone-1 vendu sous le nom commercial UVINUL 400 par BASF, la Benzophenone-2 vendu sous le nom commercial UVINUL D50 par BASF, la Benzophenone-3 ou Oxybenzone, vendu sous le nom commercial UVINUL M40 par BASF, la Benzophenone-4 vendu sous le nom commercial UVINUL MS40 par BASF ; les Dérivés du benzylidène camphre tels que le 4-Methylbenzylidene camphre vendu sous le nom commercial EUSOLEX 6300 par MERCK ; les dérivés du phenyl benzimidazole tels que le Benzimidazilate vendu sous le nom commercial NEO HELIOPAN AP par Haarmann et Reimer ; les dérivés de la triazine tels que l'Anisotriazine vendu sous le nom commercial TINOSORB S par CIBA GEIGY et l'éthylhexyl triazone vendu notamment sous le nom commercial UVINUL T150 par BASF ; les dérivés du phenyl benzotriazole tels que Drometrizole Trisiloxane vendu sous le nom commercial SILATRIZOLE par Rhodia Chimie ; les dérivés anthraniliques tels que le Menthyl anthranilate vendu sous le nom commercial NEO HELIOPAN MA par Haarmann et Reimer ; les dérivés d'imidazolines ; les dérivés du benzalmalonate ; et leurs mélanges.

Les compositions selon l'invention peuvent être préparées par tout procédé approprié. Selon un mode préféré de réalisation, les compositions de l'invention sont préparées en mélangeant d'une part les constituants aqueux ou hydrophiles et d'autre part les constituants huileux ou lipophiles formant la phase huileuse de l'émulsion, en chauffant éventuellement entre 40 et 50°C, en versant le mélange des constituants huileux dans le mélange des constituants aqueux et en agitant environ de 10 à 30 minutes, puis en laissant reposer jusqu'à déphasage en une phase inférieure aqueuse et une phase supérieure non transparente constituée d'une émulsion..

Les compositions décrites ci-dessus peuvent être conditionnées, de façon connue, dans un flacon à un seul compartiment. L'utilisateur doit alors agiter le flacon avant d'en verser le contenu sur un coton. On peut également prévoir que les deux phases de la composition soient introduites dans deux compartiments indépendants d'un même flacon, un système étant prévu pour leur mélange au moment de la distribution. De tels dispositifs sont décrits par exemple dans les documents EP-A-497256 et FR-A-2697233.

La composition selon l'invention peut être utilisée pour toute application topique ; notamment, elle peut constituer une composition cosmétique ou dermatologique. Elle peut en particulier être utilisée pour le soin, le nettoyage et/ou le démaquillage de la peau, les lèvres et /ou des yeux, et également comme composition pour le soin des cheveux.

L'invention a aussi pour objet l'utilisation cosmétique d'une composition cosmétique telle que définie ci-dessus, pour le soin, le démaquillage et/ou le nettoyage de la peau, des lèvres et/ou des yeux, et/ou pour le soin des cheveux.

La présente invention a encore pour objet un procédé cosmétique de démaquillage, de nettoyage et/ou de soin de la peau, des lèvres et/ou des yeux, caractérisé par le fait que l'on applique sur la peau, les lèvres et/ou les yeux, une composition cosmétique telle que définie ci-dessus.

La présente invention a aussi pour objet un procédé cosmétique de soin des cheveux, caractérisé par le fait que l'on applique sur les cheveux, une composition cosmétique telle que définie ci-dessus.

Les exemples ci-après de compositions selon l'invention sont donnés à titre d'illustration et sans caractère limitatif. Les quantités y sont données en % en poids, sauf mention contraire.

### Exemple 1 : composition de soin de la peau

| *Phase huileuse :* | | |
|---|---|---|
| - Cyclopentasiloxane | 7 | % |
| - Isohexadécane | 2 | % |
| - Polyisobutène hydrogéné | 8 | % |
| - conservateur lipophile | 0,4 | % |
| - Vitamine E | 0,7 | % |
| - parfum | 0,3 | % |

| *Constituants aqueux* | | |
|---|---|---|
| - Glycérine | 7 | % |
| - Butylène glycol | 4 | % |
| - Hyaluronate de sodium (hydratant) | 0,5 | % |
| - conservateur hydrophile | 0,2 | % |
| - MACKANATE DC 50 à 50 % de matière active (soit 2,5 % de Disodium PEG-12 Dimethicone sulfosuccinate) | 5 | % |
| - Eau déminéralisée qsp 100 % | | |

Mode opératoire : d'une part, on prépare le mélange des constituants aqueux et d'autre part on mélange les constituants de la phase huileuse. Puis, on verse la phase huileuse dans le mélange des constituants aqueux et on agite.

On obtient une composition qui, au repos, comporte une phase aqueuse et une émulsion distinctes. Quand on les agite, il y a formation d'une émulsion unique. Après l'arrêt de l'agitation, les deux phases se séparent rapidement, c'est-à-dire après un temps pouvant aller d'une minute à 30 minutes.

La composition biphase obtenue a de bonnes propriétés hydratantes et peut être notamment utilisée pour l'hydratation de la peau.

### Exemple 2 : composition démaquillante

| *Phase huileuse :* | | |
|---|---|---|
| - Cyclopentasiloxane | 7 | % |
| - Isohexadécane | 20 | % |
| - Polyisobutène hydrogéné | 10 | % |
| - conservateur lipophile | 0,2 | % |
| - parfum | 0,3 | % |

| *Constituants aqueux* | | |
|---|---|---|
| - Glycérine | 4 | % |
| - Butylène glycol | 6 | % |
| - conservateur hydrophile | 0,3 | % |
| - MACKANATE DC 50 à 50 % de matière active (soit 10 % de Disodium PEG-12 Dimethicone sulfosuccinate) | 20 | % |
| - Eau déminéralisée qsp 100 % | | |

Mode opératoire : d'une part, on prépare le mélange des constituants aqueux et d'autre part on mélange les constituants de la phase huileuse. On chauffe à 40-50°C ces mélanges puis on verse les constituants huileux dans le mélange aqueux, et on agite.

On obtient une composition qui, au repos, comporte une phase aqueuse et une émulsion distinctes. Quand on les agite, il y a formation d'une émulsion unique. Après l'arrêt de l'agitation, les deux phases se séparent rapidement, c'est-à-dire après un temps pouvant aller d'une minute à 30 minutes.

L'analyse des deux phases séparées montre que la partie supérieure est composée de l'émulsion (émulsion sous forme de lait) comprenant 37,5 % de phase huileuse, 20 % de tensioactif et entre 5 et 10 % de phase aqueuse, et que la phase inférieure est une phase aqueuse comprenant 4% de glycérine, 6 % de butyléne glycol, 0,3 % de conservateur, le reste étant de l'eau.

La composition biphase obtenue a de bonnes propriétés de démaquillage et peut être notamment utilisée pour le démaquillage de la peau et des yeux.

### Exemple 3 : composition démaquillante

| *Phase huileuse :* | | |
|---|---|---|
| - Cyclopentasiloxane | 4 | % |
| - Isoparaffine | 3 | % |
| - Isohexadécane | 25 | % |
| - Polyisobutène hydrogéné | 7 | % |
| - conservateur lipophile | 0,2 | % |
| - Vitamine E | 0,7 | % |
| - parfum | 0,3 | % |

| *Constituants aqueux :* | | |
|---|---|---|
| - Glycérine | 5 | % |
| - Butylène glycol | 6 | % |
| - propylène glycol | 2 | % |
| - conservateur hydrophile | 0,3 | % |
| - MACKANATE DC 50 à 50 % de matière active (soit 15 % de Disodium PEG-12 Dimethicone sulfosuccinate) | 30 | % |
| - Eau déminéralisée qsp 100 % | | |

Mode opératoire : d'une part, on prépare le mélange des constituants aqueux et d'autre part on mélange les constituants de la phase huileuse. On chauffe vers 40-50°C ces mélanges puis on verse les constituants huileux dans le mélange aqueux, et on agite.

On obtient une composition qui, au repos, comporte une phase aqueuse et une émulsion distinctes. Quand on les agite, il y a formation d'une émulsion unique. Après l'arrêt de l'agitation, les deux phases se séparent rapidement, c'est-à-dire après un temps pouvant aller d'une minute à 30 minutes.

La composition biphase obtenue a de bonnes propriétés de démaquillage et peut être notamment utilisée pour le démaquillage de la peau et des yeux.

## Revendications

1. Composition pour application topique, constituée de deux phases distinctes l'une au-dessus de l'autre, comprenant (1) une phase aqueuse inférieure, (2) une phase supérieure constituée d'une émulsion huile-dans-eau, (3) au moins un tensioactif anionique choisi parmi les sels de sodium d'esters de diméthicone copolyol et d'acide sulfosuccinique et où la phase aqueuse inférieure et la phase aqueuse de l'émulsion huile-dans-eau ont la même composition.

2. Composition selon la revendication 1, **caractérisée en ce que** le rapport pondéral entre la phase aqueuse et l'émulsion va de 25/75 à 90/10, et de préférence de 30/70 à 60/40.

3. Composition selon l'une quelconque des revendications précédentes, **caractérisée en ce que** le tensioactif choisi parmi les sels de sodium d'esters de diméthicone copolyol et d'acide sulfosuccinique est un composé de formule (I) : 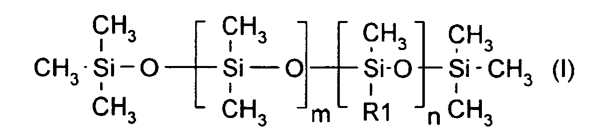 dans laquelle R₁ peut représenter : 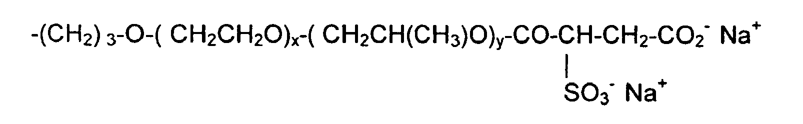 Ou bien : 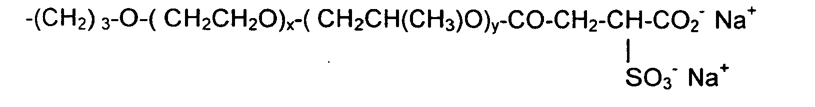 où m et n vont de 2 à 100 ; x va de 1 à 50, y va de 0 à 50.

4. Composition selon l'une quelconque des revendications précédentes, **caractérisée en ce que** le tensioactif choisi parmi les sels de sodium d'esters de diméthicone copolyol et d'acide sulfosuccinique est le PEG-12 dimethicone sulfosuccinate de disodium.

5. Composition selon l'une quelconque des revendications précédentes, **caractérisée en ce que** la quantité de tensioactif choisi parmi les sels de sodium d'esters de diméthicone copolyol et d'acide sulfosuccinique va de 0,1 à 20 % en poids, de préférence de 0,2 à 15 % en poids par rapport au poids total de la composition.

6. Composition selon l'une quelconque des revendications précédentes, **caractérisée en ce qu'**elle comprend une ou plusieurs huiles choisies parmi les huiles hydrocarbonées d'origine minérale ou synthétique et les huiles de silicone.

7. Composition selon la revendication précédente, **caractérisée en ce qu'**elle comprend au moins une huile volatile choisie parmi les iso-alcanes, les huiles de silicone volatiles, et leurs mélanges.

8. Composition selon l'une quelconque des revendications précédentes, **caractérisée en ce qu'**elle comprend une quantité globale de constituants aqueux allant de 10 à 90% en poids, de préférence de 20 à 80 % en poids par rapport au poids total de la composition.

9. Composition selon l'une quelconque des revendications précédentes, **caractérisée en ce qu'**elle constitue une composition cosmétique ou dermatologique.

10. Utilisation cosmétique d'une composition cosmétique selon l'une quelconque des revendications 1 à 8, pour le soin, le démaquillage et/ou le nettoyage de la peau, des lèvres et/ou des yeux et/ou pour le soin des cheveux.

11. Procédé cosmétique de démaquillage, de nettoyage et/ou de soin de la peau, des lèvres et/ou des yeux, **caractérisé par le fait que** l'on applique sur la peau, les lèvres et/ou les yeux, une composition cosmétique selon l'une quelconque des revendications 1 à 8.

12. Procédé cosmétique de soin des cheveux, **caractérisé par le fait que** l'on applique sur les cheveux, une composition cosmétique selon l'une quelconque des revendications 1 à 8.

## Claims

1. Composition for topical application, constituted of two separate phases one on top of the other, comprising (1) a lower aqueous phase, (2) an upper phase composed of an oil-in-water emulsion, (3) at least one anionic surfactant chosen from the sodium salts of esters of dimethicone copolyol and of sulphosuccinic acid and where the lower aqueous phase and the aqueous phase of the oil-in-water emulsion have the same composition.

2. Composition according to Claim 1, **characterized in that** the weight ratio of the aqueous phase to the emulsion ranges from 25/75 to 90/10 and preferably from 30/70 to 60/40.

3. Composition according to any one of the preceding claims, **characterized in that** the surfactant chosen from the sodium salts of esters of dimethicone copolyol and of sulphosuccinic acid is a compound of formula (I) : 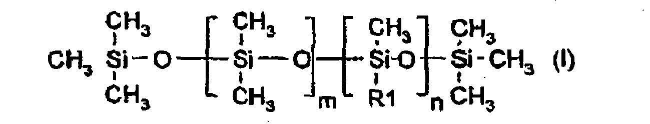 in which R₁ may represent: 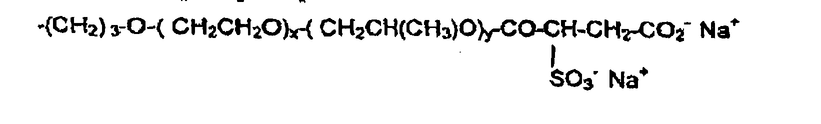 or else: 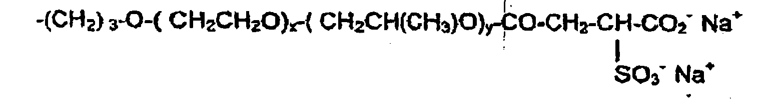 where m and n range from 2 to 100; x ranges from 1 to 50 and y ranges from 0 to 50.

4. Composition according to any one of the preceding claims, **characterized in that** the surfactant chosen from the sodium salts of esters of dimethicone copolyol and of sulphosuccinic acid is disodium PEG-12 dimethicone sulphosuccinate.

5. Composition according to any one of the preceding claims, **characterized in that** the amount of surfactant chosen from the sodium salts of esters of dimethicone copolyol and of sulphosuccinic acid ranges from 0.1 to 20% by weight, preferably from 0.2 to 15% by weight, relative to the total weight of the composition.

6. Composition according to any one of the preceding claims, **characterized in that** it comprises one or more oils chosen from hydrocarbon-based oils of mineral or synthetic origin and silicone oils.

7. Composition according to the preceding claim, **characterized in that** it comprises at least one volatile oil chosen from isoalkanes, volatile silicone oils, and mixtures thereof.

8. Composition according to any one of the preceding claims, **characterized in that** it comprises an overall amount of aqueous constituents ranging from 10 to 90% by weight, preferably from 20 to 80% by weight, relative to the total weight of the composition.

9. Composition according to any one of the preceding claims, **characterized in that** it forms a cosmetic or dermatological composition.

10. Cosmetic use of a cosmetic composition according to any one of Claims 1 to 8, for caring for, removing makeup from and/or cleansing the skin, lips and/or eyes and/or for hair care.

11. Cosmetic process for removing makeup from, cleansing and/or caring for the skin, lips and/or eyes, **characterized in that** a cosmetic composition according to any one of Claims 1 to 8 is applied to the skin, lips and/or eyes.

12. Cosmetic hair care process, **characterized in that** a cosmetic composition according to any one of Claims 1 to 8 is applied to the hair.

## Patentansprüche

1. Zusammensetzung für die topische Anwendung, die aus zwei unterschiedlichen, übereinander liegenden Phasen besteht und (1) eine untere wässrige Phase, (2) eine obere Phase, die aus einer Öl-in-Wasser-Emulsion besteht, und (3) mindestens einen anionischen grenzflächenaktiven Stoff enthält, der unter den Natriumsalzen von Estern des Dimeticoncopolyols und der Sulfobernsteinsäure ausgewählt ist, wobei die untere wässrige Phase und die wässrige Phase der Öl-in-Wasser-Emulsion die gleiche Zusammensetzung haben.

2. Zusammensetzung nach Anspruch 1, **dadurch gekennzeichnet, dass** das Gewichtsverhältnis der wässrigen Phase und der Emulsion im Bereich von 25/75 bis 90/10 und vorzugsweise im Bereich von 30/70 bis 60/40 liegt.

3. Zusammensetzung nach einem der vorhergehenden Ansprüche, **dadurch gekennzeichnet, dass** der grenzflächenaktive Stoff, der unter den Natriumsalzen von Estern des Dimeticoncopolyols und der Sulfobernsteinsäure ausgewählt ist, eine Verbindung der folgenden Formel (1) ist: 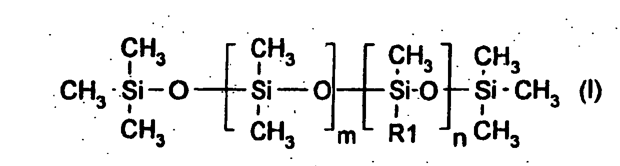 wobei R₁ bedeuten kann: 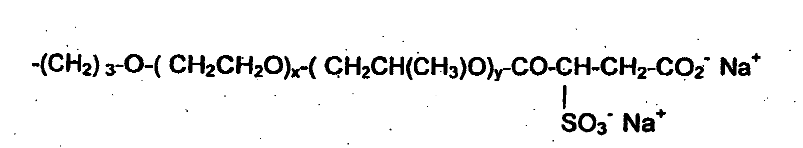 oder: 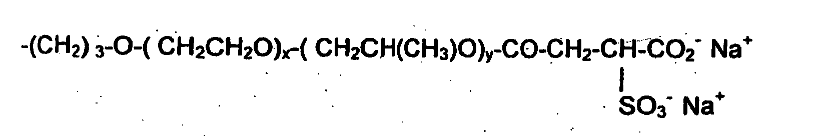 wobei m und n im Bereich von 2 bis 100 liegen; x im Bereich von 1 bis 50 liegt und y im Bereich von 0 bis 50 liegt.

4. Zusammensetzung nach einem der vorhergehenden Ansprüche, **dadurch gekennzeichnet, dass** der grenzflächenaktive Stoff, der unter den Natriumsalzen von Estern des Dimeticoncopolyols und der Sulfobernsteinsäure ausgewählt ist, das Disodium PEG-12 Dimethicone Sulfosuccinate ist.

5. Zusammensetzung nach einem der vorhergehenden Ansprüche, **dadurch gekennzeichnet, dass** der Mengenanteil des grenzflächenaktiven Stoffes, der unter den Natriumsalzen von Estern des Dimeticoncopolyols und der Sulfobernsteinsäure ausgewählt ist, im Bereich von 0,1 bis 20 Gew.-% und vorzugsweise im Bereich von 0,2 bis 15 Gew.-%, bezogen auf das Gesamtgewicht der Zusammensetzung, liegt.

6. Zusammensetzung nach einem der vorhergehenden Ansprüche, **dadurch gekennzeichnet, dass** sie ein oder mehrere Öle enthält, die unter den Kohlenwasserstoffölen mineralischer oder synthetischer Herkunft und Siliconölen ausgewählt sind.

7. Zusammensetzung nach dem vorgehenden Anspruch, **dadurch gekennzeichnet, dass** sie mindestens ein flüchtiges Öl enthält, das unter den Isoalkanen, flüchtigen Siliconölen und deren Gemischen ausgewählt ist.

8. Zusammensetzung nach einem der vorhergehenden Ansprüche, **dadurch gekennzeichnet, dass** sie eine Gesamtmenge an wässrigen Bestandteilen im Bereich von 10 bis 90 Gew.-% und vorzugsweise von 20 bis 80 Gew.-%, bezogen auf das Gesamtgewicht der Zusammensetzung, enthält.

9. Zusammensetzung nach einem der vorhergehenden Ansprüche, **dadurch gekennzeichnet, dass** es sich um eine kosmetische oder dermatologische Zusammensetzung handelt.

10. Kosmetische Verwendung einer kosmetischen Zusammensetzung nach einem der Ansprüche 1 bis 8 für die Pflege, zum Abschminken und/oder für die Reinigung der Haut, der Lippen und/oder der Augen und/oder für die Pflege der Haare.

11. Kosmetisches Verfahren zum Abschminken, Reinigen und/oder Pflegen der Haut, der Lippen und/oder der Augen, **dadurch gekennzeichnet, dass** auf die Haut, die Lippen und/oder die Augen eine kosmetische Zusammensetzung nach einem der Ansprüche 1 bis 8 aufgetragen wird.

12. Kosmetisches Verfahren für die Pflege der Haare, **dadurch gekennzeichnet, dass** auf die Haare eine kosmetische Zusammensetzung nach einem der Ansprüche 1 bis 8 aufgetragen wird.
